(19) Europäisches Patentamt
European Patent Office
Office européen des brevets

(11) Veröffentlichungsnummer : **0 346 788 B1**

# (12) EUROPÄISCHE PATENTSCHRIFT

(45) Veröffentlichungstag der Patentschrift : **08.04.92 Patentblatt 92/15**

(51) Int. Cl.$^5$ : **C07C 271/42,** C08F 220/18, C08G 18/00

(21) Anmeldenummer : **89110564.5**

(22) Anmeldetag : **10.06.89**

## (54) Strahlungsempfindliche, ethylenisch ungesättigte, copolymerisierbare Verbindungen und Verfahren zu deren Herstellung.

(30) Priorität : **16.06.88 DE 3820463**

(43) Veröffentlichungstag der Anmeldung : **20.12.89 Patentblatt 89/51**

(45) Bekanntmachung des Hinweises auf die Patenterteilung : **08.04.92 Patentblatt 92/15**

(84) Benannte Vertragsstaaten : **AT BE CH DE ES FR GB IT LI NL SE**

(56) Entgegenhaltungen : **EP-A- 0 246 848**

(56) Entgegenhaltungen :
**DE-A- 2 031 477**
**US-A- 3 322 818**
**US-A- 3 429 852**
**US-A- 4 634 791**

(73) Patentinhaber : **BASF Aktiengesellschaft**
**Carl-Bosch-Strasse 38**
**W-6700 Ludwigshafen (DE)**

(72) Erfinder : **Boettcher, Andreas, Dr.**
**Konrad-Adenauer-Ring 38**
**W-6907 Nussloch (DE)**
Erfinder : **Rehmer, Gerd, Dr.**
**Koenigsberger Strasse 1**
**W-6712 Bobenheim-Roxheim (DE)**

**EP 0 346 788 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neuartige strahlungsempfindliche, ethylenisch ungesättigte Aceto- und Benzophenonderivate und ein Verfahren zu ihrer Herstellung.

UV-lichtempfindliche Aceto- und Benzophenone werden häufig als sog. äußere Initiatoren strahlungsempfindlichen Polymeren zugesetzt (z.B. G. Li Bassi, J. Rad. Cur. 14, 18 (1987)). Im allgemeinen sind solche Arbeitsweisen aber nicht vollständig befriedigend, denn es treten nach Mischung mit dem Polymeren Probleme mit der Verträglichkeit, der Gleichförmigkeit der Verteilung, der Flüchtigkeit, des Geruches, der Toxizität, des Ausschwitzens und der Wanderung des Zusatzstoffes auf, die häufig zu einer unerwünschten, vorzeitigen und ungleichmäßigen Reaktion führen. Beim eigentlichen Belichtungsvorgang wird dann eine geringere Reaktivität aufgrund erniedrigter effektiver Initiatorkonzentrationen beobachtet.

Es ist bekannt, daß eine Reihe der genannten Probleme gelöst werden kann, wenn der strahlungsempfindliche Initiator mit Monomeren nach einem üblichen Verfahren mischpolymerisiert, d.h. in eine Polymerkette eingebaut wird. Der lichtempfindliche Photoinitiator hängt mit einer Ankergruppe, dem sog. Spacer, am Basispolymeren. Der Spacer dient weiterhin dazu, den Einfluß der Basispolymerkette auf das photochemische Verhalten des Initiators zu reduzieren.

Copolymerisierbare Initiatoren haben daher prinzipiell folgenden Aufbau:

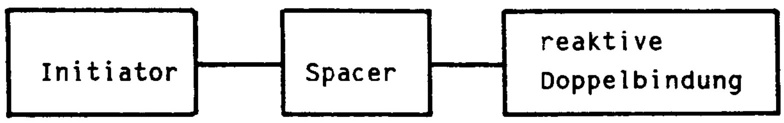


Schema I

In den US-Patentschriften 32 14 492 und 34 29 852 sind mit Acryloxy- oder Methacryloxygruppen substituierte Aceto- und Benzophenonderivate, wie z.B.

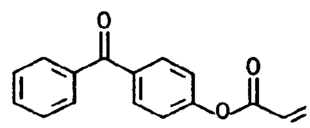

beschrieben. Diese können mit Ethylen oder anderen Vinylmonomeren mischpolymerisiert werden. Dabei entstehen Polymere, die beispielsweise nach der thermischen Verformung durch Bestrahlung gehärtet werden. Im Rahmen des Modells nach Schema I werden bei diesen strahlungsempfindlichen Monomeren die reaktive Doppelbindung und der Spacer durch die Acryloxygruppe repräsentiert.

Beim 4-(4'-Vinylbenzyloxy)benzophenon,

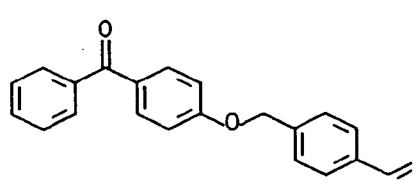

beschrieben in der DE-A-28 18 763, übernimmt der Styrylbenzyloxyrest, diese Funktion.

Beim "Uvecryl® P36", einem Handelsprodukt der Fa. UCB, trennt ein besonders langer Spacer aus vier Ethylenoxyeinheiten das Benzophenon vom Acryloxy-Rest.

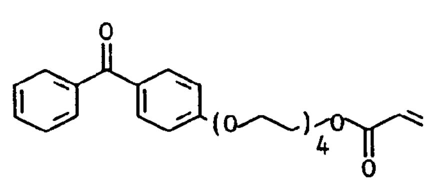

Diese z.B. im Technical Bulletin 2480/885 (1985) der UCB beschriebene Verbindung kann in Photopolymeren für Überzugsmassen eingesetzt werden. Die Synthese ist aufwendig.

Der oben genannten Patentliteratur ist zu entnehmen, daß die Ether- oder Estergruppe ein wichtiger Bestandteil des Spacers ist (siehe Schema II).

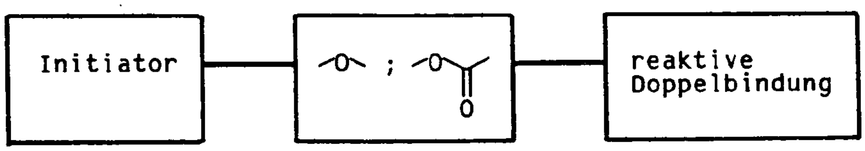


Schema II

Die Ether- bzw. Estergruppen dienen dabei vorwiegend als Verknüpfungselemente zwischen Spacer und Initiatorfragment.

Zu einem neuen Strukturschema gelangt man, indem man den Einfluß des Spacers als Substituenten auf das photochemisch angeregte Aceto- bzw. Benzophenonfragment betrachtet. So sind beispielsweise Spacer denkbar, die aufgrund ihrer Struktur stabilisierend oder destabilisierend wirken können.

Insbesondere die carbamoylsubstituierten Benzophenone des Typs

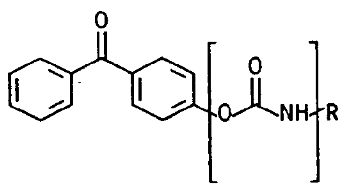

Schema III

stellen unter diesem Gesichtspunkt eine interessante Substanzklasse dar. In der US-PS 33 22 818 werden allyl- bzw. methallyl-substituierte Carbamoylbenzophenone beschrieben. Sie eignen sich aber nur als Fungizide (vgl. Schema IV).

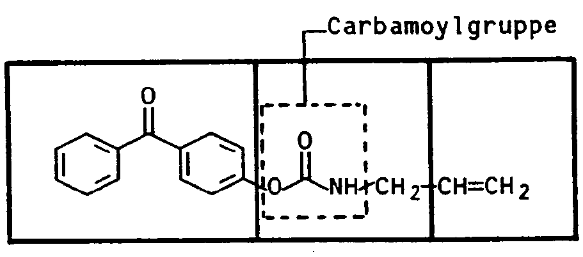


Initiator Spacer Doppelbindung

Schema IV

Für Copolymerisationen ist jedoch eine Allyl- bzw. Methallylgruppe nicht geeignet. Diese Benzoylphenyl-allyl-carbamate haben daher im Polymerbereich keine praktische Bedeutung.

In noch stärkerem Maße gilt dies für die in der DE-A-20 31 477 beschriebenen UV-Stabilisatoren für Polyurethane. Diese Verbindungen enthalten zwar im Spacer eine Carbamoylgruppe, dafür aber keine reaktive Doppelbindung.

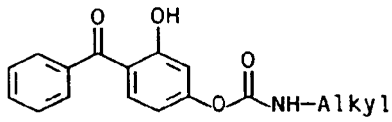

Eine photochemisch interessante Strukturvariante wird in der EP-A-0 246 848 beschrieben. Durch Umsetzung eines reaktiven Isocyanats mit Hydroxyethyloxy-benzophenon erhält man ein strahlungsempfindliches Monomeres mit einem besonders langen Spacer.

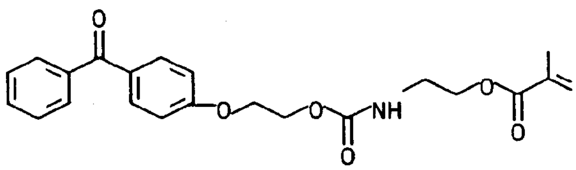

Dieses Monomere ist copolymerisierbar, hat aber den entscheidenden Nachteil, daß der strahlungsempfindliche Teil des Moleküls über eine photochemisch nur schwach aktivierende Ethergruppe mit dem Spacer verbunden ist.

Aufgabe der vorliegenden Erfindung ist es, ein strahlungsempfindliches, ethylenisch ungesättigtes Aceto- bzw. Benzophenonderivat eines neuen, bisher nicht bekannten Typs aufzuzeigen, bei dem die Carbamoylgruppe direkt an den Initiatorteil gebunden ist und das zusätzlich eine reaktive copolymerisierbare C-C-Doppelbindung enthält.

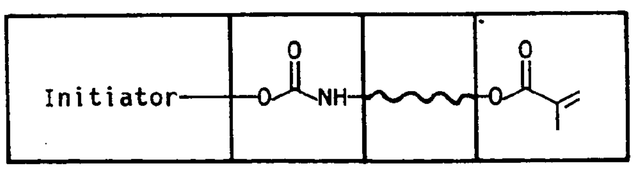


Schema V

Gegenstand der Erfindung sind ethylenisch ungesättigte copolymerisierbare, strahlungsempfindliche organische Verbindungen der allgemeinen Formel (I),

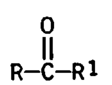

(I),

worin

R für einen geradkettigen Alkylrest mit 1 bis 4 Kohlenstoffatomen, vorzugsweise Methyl, Ethyl, n-Propyl, einen verzweigten, gegebenenfalls substituierten Alkylrest mit 3 oder 4 Kohlenstoffatomen, wie iso-Propyl, sek.-Hydroxyisopropyl, tert.-Butyl, einen Arylrest, wie Phenyl, Tolyl oder Naphthyl, oder den Rest $R^1$ steht und $R^1$ für den Rest

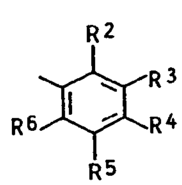

steht, wobei die Reste $R^2$ bis $R^6$ untereinander gleich oder verschieden sind und für H, Alkyl mit 1 bis 3 Kohlenstoffatomen, z.B. Methyl, Ethyl, n-Propyl, iso-Propyl, OH, $OCH_3$, $OC_2H_5$, SH, $SCH_3$, $SC_2H_5$, Cl, F, $N(CH_3)_2$, $N(C_2H_5)_2$ oder $N(CH_3)C_6H_5$ stehen und mindestens einer aber maximal drei der Reste $R^2$ bis $R^6$ für den Rest

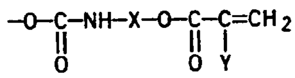

stehen, worin X für einen zweiwertigen Alkylenrest-$(CH_2)_m$- mit m = 1 bis 10, vorzugsweise Ethylen, einen perfluorierten Alkylenrest-$(CF_2)_m$- mit m = 1 bis 10, vorzugsweise Perfluorethylen, einen Oxaalkylenrest des Typs -$(CH_2)_n$-O-$(CH_2)_p$- mit n = 1 bis 5 und p = 1 bis 5, vorzugsweise n = p = 2, d.h. -$C_2H_4$-O-$C_2H_4$-, einen perfluorierten Oxaalkylenrest des Typs -$(CF_2)_n$-O-$(CF_2)_p$- mit n = p = 1 bis 5, beispielsweise Tetrafluorethylen, oder einen gegebenenfalls perfluorierten Polyoxaalkylenrest mit 2 bis 5 Sauerstoffatomen, die miteinander über mindestens eine -$CH_2$- oder $CF_2$-Gruppe verbunden sind und Y für H oder Methyl stehen.

Überraschenderweise zeigen die erfindungsgemäßen Verbindungen eine besonders hohe photochemische Reaktivität im mittel- bis längerwelligen UV-Bereich.

Eine weitere Aufgabe der vorliegenden Erfindung bestand darin, ein Verfahren zur Herstellung neuartiger strahlungsempfindlicher Carbamoylbenzo- und -acetophenone mit mindestens einer Methacrylat- bzw. Acrylatendgruppe aufzuzeigen.

Die Synthese von Arylcarbamaten ohne copolymerisationsfähige Endgruppe ist bekannt. Eine gute übersicht gibt C. Ferri, "Reaktionen der organischen Synthese", G. Thieme Verlag, Stuttgart, 1978.

Das wichtigste Darstellungsverfahren ist die Umsetzung von aromatischen Alkoholen mit Isocyanaten (vgl. Houben-Weyl VIII, S. 141; 0.S. Petersen, Liebigs Ann. Chem. 562, 205 (1947); J. Burkus, 3. Org. Chem. 26, 779 (1961); I.T. Kay und N. Punja, J. Chem. Soc. [C] 1968, 3011; L. Capuano und R. Zander, Chem. Ber. 104, 2212 (1971)). Die Carbamate entstehen in guten bis sehr guten Ausbeuten, wenn der Alkohol und das Isocyanat im Molverhältnis 1:1 ohne Solvens oder in überschüssigem Alkohol als Lösungsmittel miteinander zur Reaktion gebracht werden. In den Fällen, in denen der Alkohol bzw. das Phenol als Feststoff vorliegen, verwendet man aprotische Lösungsmittel, wie z.B. Dichlormethan, Dichlorethan, Acetonitril, Toluol usw.

Bei der Übertragung dieses Darstellungsverfahrens auf ω-Isocyanatoalkyl(meth)acrylate der allgemeinen-Formel (II), in der X für einen gegebenenfalls perfluorierten Alkylenrest, einen Oxaalkylenrest oder einen Polyoxaalkylenrest mit jeweils 2 bis 12 C-Atomen und Y für H- oder $CH_3$- stehen, wurde überraschend gefunden, daß die gewünschten Carbamoylbenzophenone mit (Meth)Acrylatgruppen in hoher, fast quantitativer Ausbeute entstehen. Dies ist insofern überraschend, als Acrylate bzw. Methacrylate leicht zahlreiche Nebenreaktionen (Vernetzung, Polymerisation) eingehen können.

Die für die Umsetzung der Hydroxyaceto- bzw. -benzophenone benötigten ω-Isocyanatoalkyl(meth)-acrylate sind in guter Ausbeute nach dem in den Patentschriften EP-A-083764 und DE-A-35 23 692 beschriebenen Verfahren herstellbar.

Die als weiteres Ausgansmaterial benötigten Hydroxyacetophenone und Hydroxybenzophenone sind nach bekannten Verfahren herstellbar. So erhält man beispielsweise 4-Hydroxybenzophenon in ca. 90 %iger Ausbeute durch Friedel-Crafts-Acylierung von Phenol mit Benzoylchlorid in Nitrobenzol in Gegenwart von $AlCl_3$ oder $TiCl_4$ (Houben-Weyl 7/2a, S. 186) oder isomerenfrei durch Oxidation von 4-Hydroxy-diphenylmethan mit 5,6-Dichlor-2,3-dicyan-p-benzochinon (Houben-Weyl 7/2a, S. 681).

Gegenstand der vorliegenden Erfindung ist außerdem ein Verfahren zur Herstellung von Verbindungen der allgemeinen Formel (I), wobei man eine Verbindung der allgemeinen Formel (II)

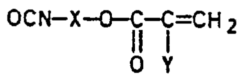

(II),

worin X und Y die oben angegebene Bedeutung haben, vorzugsweise wäre dies z.B. Isocyanatoethylmethacrylat oder 5-Isocyanato-3-oxapentylmethacrylat, mit einer Verbindung der allgemeinen Formel (III)

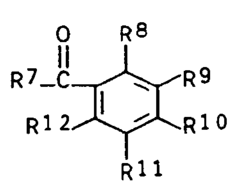

(III),

worin $R^7$ für einen geradkettigen Alkylrest mit 1 bis 4 Kohlenstoff-atomen, vorzugsweise Methyl, Ethyl, n-Propyl,

einen verzweigten, gegebenenfalls substituierten Alkylrest mit 3 bis 4 Kohlenstoffatomen, wie i-Propyl, sek.-Hydroxyisopropyl, t-Butyl, oder einen Arylrest, z.B. Phenyl, Tolyl oder Naphthyl, vorzugsweise Phenyl steht und worin die Reste $R^8$ bis $R^{12}$ untereinander gleich oder verschieden sind und für H, Alkyl mit 1 bis 3 Kohlenstoffatomen, z.B. Methyl, Ethyl, n-Propyl, i-Propyl, OH, $OCH_3$, $OC_2H_5$, SH, $SCH_3$, $SC_2H_5$, Cl, F, $N(CH_3)_2$, $N(C_2H_5)_2$ oder $N(CH_3)C_6H_5$ stehen mit der Maßgabe, daß mindestens einer der Reste $R^8$ bis $R^{12}$ für eine Hydroxylgruppe steht, im äquimolaren Verhältnis oder dem Zwei- oder Dreifachen davon, u.U. mit einem max. 20 %igen Überschuß des Isocyanats, unter Feuchtigkeitsausschluß, gegebenenfalls in Gegenwart eines inerten Lösungsmittels oder Lösungsmittelgemisches und eines basischen Katalysators bei Temperaturen von 0 bis 100, vorzugsweise bei 20 bis 50°C, miteinander umsetzt.

Zum Herstellverfahren ist im einzelnen Folgendes auszuführen.

Die bei der Reaktion eingesetzten Isocyanate reagieren leicht mit Nucleophilen, u.a. auch mit Wasser. Deshalb ist bei der Reaktion auf strikten Feuchtigkeitsausschluß durch Verwendung getrockneter nicht nucleophiler Lösungsmittel, wie z.B. Acetonitril, Dichlormethan, Dichlorethan, THF, Toluol, Chlorbenzol, Essigester, Chloroform usw. und den Aufbau einer Inertgasatmosphäre, z.B. Stickstoff, Argon oder Kohlendioxid zu achten.

In der Regel wird eine Lösung der Hydroxyverbindung in einem inerten Lösungsmittel, welches auch wegfallen kann, wenn die Verbindung der allgemeinen Formel (III) flüssig ist, bei Temperaturen von 0 bis 100°C, vorzugsweise bei 20 bis 50°C, vorgelegt. Dann wird das flüssige Isocyanat unter Rühren zugetropft. Zu dieser Mischung kann dann zum Starten der Reaktion ein basisches, nicht nucleophiles Amin, vorzugsweise Triethylamin, 4-Dimethylaminopyridin, 1,4-Diazabicyclo[2.2.2]octan, 1,5-Diazabicyclo[4.3.0]non-5-en, 1,8-Diazabicyclo[5.4.0]undec-7-en, Polyvinylpyridin usw., zweckmäßigerweise gelöst in einem inerten organischen Lösungsmittel, so zugetropft werden, daß die Innentemperatur unter 100°C, vorzugsweise bei 20 bis 50°C gehalten werden kann.

Diese Arbeitsweise eignet sich hervorragend für Laboransätze.

Für Arbeiten im größeren Maßstab führt man die Synthese vorzugsweise in der folgenden leicht modifizierten Weise durch.

In einem inerten Lösungsmittel werden Verbindungen der allgemeinen Formel (III) und das Amin unter Feuchtigkeitsausschluß bei Temperaturen von 20 bis 60°C vorgelegt und das Isocyanat, vorzugsweise ohne Lösungsmittel verdünnt, so zugetropft, daß die Innentemperatur in dem angegebenen Bereich gehalten werden kann.

Nach einer Nachrührzeit von 1 bis 20 Stunden bei 10 bis 30°C wird das Lösungsmittel im Vakuum bis zur Gewichtskonstanz abdestilliert. Kristalline Produkte lassen sich zusatzlich bequem aus Alkoholen, z.B. Isopropanol umkristallisieren.

Für alle in den folgenden Beispielen angegebenen Verbindungen wurde die Struktur durch $^1H$-NMR-, IR- und Massenspektren bestätigt.

Beispiel 1

p-N-(Methacryloylethyl)carbamoyloxy-acetophenon

In einem Vierhalskolben mit Rührer, Rückflußkühler, Innenthermometer und Tropftrichter werden bei Raumtemperatur (RT) 20 ml Toluol vorgelegt und darin 3,5 g (25 mmol) 4-Hydroxyacetophenon sowie 4,25 g (27,5 mmol) Isocyanatoethylmethacrylat gelöst. Bei einer Innentemperatur von 20 - 26°C werden dann 0,2 g (2 mmol) Triethylamin in 10 ml Tetrahydrofuran zugetropft und 2 Stunden bei RT nachgerührt. Nach der Zugabe einer kleineren Menge Phenothiazin wurde das Lösungsmittel im Wasserstrahlvakuum abdestilliert und der Rückstand aus Isopropanol umkristallisiert.
Ausbeute: 6,1 g (84 %) fast farblose Kristalle
Schmp. 87 - 88°C

Beispiel 2

p-N- (Methacryl-4-oxa-pentamethylen)carbamoyloxy-acetophenon

Die Synthese wurde in Analogie zu der in Beispiel 1 gegebenen Vorschrift durchgeführt. Folgende Mengen wurden eingesetzt:
3,4 g (25 mmol) 4-Hydroxyacetophenon
20 ml Toluol
5,5g (27,5 mmol) 5-Isocyanato-3-oxapentyl-methacrylat
0,2g (2 mmol) Triethylamin

Ausbeute: 8,2 g (98 %) gelbliches Öl

Beispiel 3

p-N- (Methacryloyl-4-oxa-pentamethylen)-carbamoyloxy-benzophenon

Methode 1

109,5 g (0,55 mol) 5-Isocyanato-3-oxa-pentamethylenmethacrylat und 99 g (0,5 mol) 4-Hydroxybenzophenon wurden bei RT in 90 ml Toluol vorgelegt und bei 20 - 25°C (schwache Kühlung erforderlich) eine Lösung von 2,1 g Triethylamin in 75 ml Tetrahydrofuran langsam zugetropft. Dabei stieg die Innentemperatur auf ca. 40°C an. Nach 12 stündigem Stehenlassen wurde die auf RT abgekühlte Reaktionsmischung mit 100 mg Phenothiazin versetzt und am Rotationsverdampfer im Ölpumpenvakuum bei max. 35°C Badtemperatur bis zur Gewichtskonstanz eingeengt.
Ausbeute: 197 g (quantitativ) honigfarbenes, viskoses Öl

Methode 2

In einer Mischung aus 90 l wasserfreiem Toluol und 125 l trockenem Tetrahydrofuran werden bei 20 bis 30°C 90 kg (0,5 kmol) 4-Hydroxybenzophenon gelöst. Anschließend werden unter Rühren 3,5 kg Triethylamin dazugegeben. Bei einer Innentemperatur von 20 bis 25°C werden 109,5 kg (0,55 kmol) 5-Isocyanato-3-oxa-pentyl-methacrylat so zudosiert, daß die Innentemperatur 30°C nicht übersteigt. Nach 12 stündigem Nachrühren bei 20 bis 25°C werden 100 g Phenothiazin zugesetzt und das Lösungsmittelgemisch bei einer maximalen Badtemperatur von 35°C bei ca. 10 bis 15 mbar abdestilliert.
Ausbeute: 198 kg (quantitativ)

Beispiele 4 - 8

In Analogie zu dem im Beispiel 3 angegebenen Vorschriften lassen sich weitere funktionalisierte Isocyanate mit Hydroxybenzophenonen umsetzen. Beispielhaft seien die folgenden Reaktionen angeführt:

| Beispiel | Isocyanat (1,2 mol) | Benzophenon (1 mol) | Ausbeute [%] | Schmp. |
|---|---|---|---|---|
| 4 | 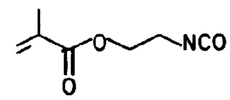 | 4-Hydroxy-benzophenon | 81 | 101 - 102°C |
| 5 | 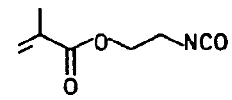 | 2-Hydroxy-benzophenon | 98 | gelbes Öl |
| 6 | 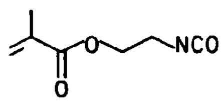 | 2,4-Dihydroxy-benzophenon | 85* | gelbliches Öl |
| 7 | 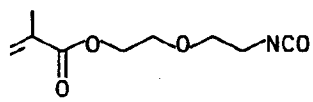 | 2-Hydroxy-benzophenon | 98 | gelbes Öl |
| 8 | 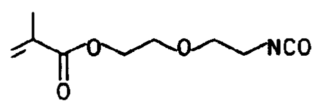 | 2,4-Dihydroxy-benzophenon | 90* | gelbes Öl |

* es reagiert ausschließlich die 4-Hydroxygruppe

Auch im Aceto- bzw. Benzophenonteil der Carbaminsäureester sind Substituenten-variationen möglich.

Beispiel 9

2-Acetyl-4-ethyl-7-methoxy-1-N-(methacryloylethylen)carbaminsäurenaphthylester

In 50 ml trockenem Toluol wurden 12,2 g (50 mmol) 2-Acetyl-4-ethyl-7-methoxy-1-naphthol und 8,6 g (55 mmol) Isocyanatoethylmethacrylat vorgelegt und innerhalb von 5 Minuten 0,5 g Triethylamin in 15 ml Tetrahydrofuran zugetropft. Nach 52-stündigem Rühren bei RT wurden die ausgefallenen Kristalle abgesaugt, mit Toluol gewaschen und im Vakuum getrocknet.
Ausbeute: 11 g (53 %) farblose Kristalle
Schmp. : 124 - 126°C (Zers.)

Beispiel 10

In Analogie zur Synthese der im Beispiel 9 beschriebenen Verbindung konnte der 4-Carbaminsäureester des 3-Benzoyl-4-hydroxy-6-methoxy-1-naphthylessigsäuremethylesters durch Umsetzung mit Isocyanatoethylmethacrylat in 64 % Ausbeute hergestellt werden.
Schmp. 147 - 148°C (Zers.)

Beispiel 11

4,4'-Bis((N-methacryloxyethyl)-carbamoyloxy)-benzophenon

In 300 ml Toluol wurden unter Feuchtigkeitsausschluß 34,2 g (0,22 mol) Isocyanatoethylmethacrylat und 22 g (0,1 mol) 4,4'-Bishydroxybenzophenon gelöst und bei RT eine Lösung von 0,7 g Triethylamin in 50 ml Tetrahydrofuran zugetropft. Die exotherm verlaufende Reaktion wurde durch Kühlung im Eisbad auf 28°C -

30°C gehalten und 10 Stunden bei RT nachgerührt. Die ausgefallenen Kristalle wurden mit Toluol nachgewaschen, getrocknet und aus Isopropanol umkristallisiert.
Ausbeute: 43 g (82 %) fast farblose Kristalle
vom Schmp. 135 - 136°C

Beispiel 12

Wie im Beispiel 11 beschrieben, kann auch das weniger reaktive 4,4′-Bis(p-hydroxy-phenylenoxy)benzophenon in den Carbaminsäureester umgewandelt werden. Man beobachtet keine exotherme Reaktion und arbeitet den Reaktionsansatz erst nach 72-stündigem Stehen bei RT auf. Das Toluolfiltrat wird im Ölpumpenvakuum eingedampft und der Rückstand aus Isopropanol umkristallisiert.
Ausbeute: 64 %
Schmp. ab 140°C Zers. (vollständige Zersetzung bei 270°C)

Analyse (Beispiel 12):

$C_{39}H_{36}N_2O_{11}$

| | | | |
|---|---|---|---|
| Ber. | C 66.1 | H 5.09 | N 3.95 |
| Gef. | 66.2 | 5.2 | 3.9 |

Beispiel 13

N-(Benzoyl-p-phenylen)-N′-(methacryloxyethylen)-carbodiimid

In 50 ml Toluol wurden unter Feuchtigkeitsausschluß 8,5 g (55 mmol) Isocyanatoethylmethacrylat und 9,8 g (50 mmol) 4-Aminobenzophenon vorgelegt. Nach Zugabe von 0,7 g Triethylamin in 15 ml Tetrahydrofuran wurde die Mischung 15 Stunden bei 55°C gerührt und nach dem Abkühlen im Wasserstrahlvakuum eingedampft. Der ölige Rückstand wurde mit Aceton/Petrolether angerieben und nach dem Kristallisieren aus Isopropanol unter Zusatz einer geringen Menge Phenothiazin umkristallisiert.
Ausbeute: 12 g (68 %) farblose Kristalle
vom Schmp. 134 - 137°C

Beispiel 14

4′-Dimethylamino-4-(N-(methacryloylethyl)-carbamoyloxy)-benzophenon

Zu der Lösung von 11,6 g (50 mmol) 4-Hydroxy-4′-dimethylaminobenzophenon und 8,6 g (55 mmol) Isocyanatoethylmethacrylat in 50 ml Toluol wurden bei bis 20°C 0,35 g Triethylamin, gelöst in 13 ml Tetrahydrofuran, zugetropft. Man beobachtete einen leichten Temperaturanstieg auf etwa 30°C. Nach 10 stündigem Rühren bei RT wurden die ausgefallenen Kristalle abgesaugt, mit Toluol nachgewaschen und aus Isopropanol umkristallisiert.
Ausbeute: 16,5 g (83 %) farblose Kristalle
vom Schmp. 127 - 130°C

Beispiel 15

4-Dimethylamino-4′-(N-(methacryloyl-4-oxa-pentamethylen)-carbamoyloxy)benzophenon

Die Verbindung wurde in Analogie zu Beispiel 12 hergestellt und eine entsprechende Menge von 11 g (55 mmol) 5-Isocyanato-3-oxa-pentyl-methacrylat eingesetzt.
Ausbeute: 22 g (quantitativ) schwach gelbes Öl

**Patentansprüche**

1. Ethylenisch ungesättigte copolymerisierbare, strahlungsempfindliche organische Verbindungen der all-

gemeinen Formel (I)

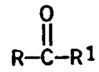

(I),

worin
R für einen geradkettigen Alkylrest mit 1 bis 4 Kohlenstoffatomen, einen verzweigten, gegebenenfalls substituierten, Alkylrest mit 3 oder 4 Kohlenstoffatomen, für einen Arylrest oder den Rest $R^1$ steht und
$R^1$ für den Rest

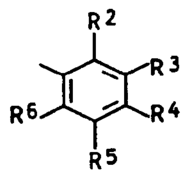

steht, wobei die Reste $R^2$ bis $R^6$ untereinander gleich oder verschieden sind und für H, Alkyl mit 1 bis 3 Kohlenstoffatomen, OH, $OCH_3$, $OC_2H_5$, SH, $SCH_3$, $SC_2H_5$, Cl, F, $N(CH_3)_2$, $N(C_2H_5)_2$ oder $N(CH_3)C_6H_5$ stehen und mindestens einer aber maximal drei der Reste $R^2$ bis $R^6$ für den Rest

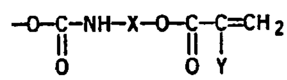

stehen, worin X für einen zweiwertigen, gegebenenfalls perfluorierten, Alkylenrest-$(CH_2)_m$- bzw. $(CF_2)_m$- mit m = 1 bis 10, einen, gegebenenfalls perfluorierten, Oxaalkylenrest des Typs-$(CH_2)_n$-O-$(CH_2)_p$- oder -$(CF_2)_n$-O-$(CF_2)_p$- mit n = 1 bis 5 und p = 1 bis 5 oder einen, gegebenenfalls perfluorierten, Polyoxaalkylenrest mit 2 bis 5 Sauerstoffatomen, die miteinander über mindestens eine -$CH_2$-oder -$CF_2$-Gruppe verbunden sind und Y für H oder Methyl stehen.

2. Verfahren zur Herstellung von Verbindungen der allgemeinen Formel (I), dadurch gekennzeichnet, daß man eine Verbindung der allgemeinen Formel (II)

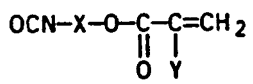

(II),

worin X und Y die in Anspruch 1 angegebene Bedeutung haben, mit einer Verbindung der allgemeinen Formel (III)

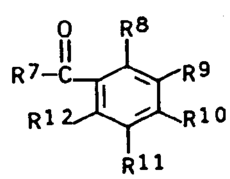

(III),

worin $R^7$ für einen geradkettigen Alkylrest mit 1 bis 4 Kohlenstoffatomen, einen verzweigten, gegebenenfalls substituierten, Alkylrest mit 3 oder 4 Kohlenstoffatomen oder einen Arylrest steht und worin die Reste $R^8$ bis $R^{12}$ untereinander gleich oder verschieden sind und für H, Alkyl mit 1 bis 3 Kohlenstoffatomen, OH, $OCH_3$, $OC_2H_5$, SH, $SCH_3$, Cl, F, $N(CH_3)_2$, $N(C_2H_5)_2$ oder $N(CH_3)C_6H_5$ stehen, mit der Maßgabe, daß mindestens einer der Reste $R^8$ bis $R^{12}$ für eine Hydroxylgruppe steht, im äquimolaren Verhältnis oder dem Zwei- oder Dreifachen davon, gegebenenfalls in Gegenwart eines inerten Lösungsmittels oder Lösungsmittelgemisches und eines basischen Katalysators, bei Temperaturen von 0 bis 100°C unter wasserfreien Bedingungen miteinander umsetzt.

3. Verfahren nach Anspruch 2, dadurch gekennzeichnet, daß es sich bei den Verbindungen der Formel (II) um ω-Isocyanatoalkyl-(meth)-acrylate handelt.

4. Verfahren nach einem der Ansprüche 2 oder 3, dadurch gekennzeichnet, daß pro Mol der Verbindung der allgemeinen Formel (III) 1 bis 1,2 Mol des Isocyanates der allgemeinen Formel (II) umgesetzt werden.

5. Verfahren nach einem der Ansprüche 2 bis 4, dadurch gekennzeichnet, daß mindestens eine katalytische Menge einer starken, nicht nucleophilen Base, vorzugsweise eines Amins, anwesend ist.

6. Verfahren nach einem der Ansprüche 2 bis 5, dadurch gekennzeichnet, daß die Reaktionstemperatur im Bereich von 20 bis 50°C liegt.

7. Verfahren nach einem der Ansprüche 2 bis 6, dadurch gekennzeichnet, daß in einem inerten, wasserfreien Lösungsmittel unter Feuchtigkeitsausschluß gearbeitet wird.

## Claims

1. An ethylenically unsaturated copolymerizable, radiation-sensitive organic compound of the formula (I)

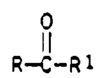

(I),

where R is a straight-chain alkyl radical of 1 to 4 carbon atoms, an unsubstituted or substituted, branched alkyl radical of 3 or 4 carbon atoms, aryl or a radical $R^1$, and $R^1$ is a radical

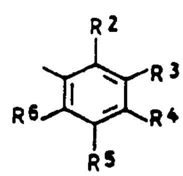

where $R^2$ to $R^6$ are identical or different and are each H, alkyl of 1 to 3 carbon atoms, OH, $OCH_3$, $OC_2H_5$, SH, $SCH_3$, $SC_2H_5$, Cl, F, $N(CH_3)_2$, $N(C_2H_5)_2$ or $N(CH_3)C_6H_5$, and not less than one but not more than three of the radicals $R^2$ to $R^6$ are each a radical

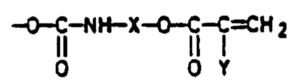

where X is a divalent alkylene radical $-(CH_2)_m-$ or $-(CF_2)_m-$which may be perfluorinated and in which m is from 1 to 10, an oxaalkylene radical of the type $-(CH_2)_n-O-(CH_2)_p-$ or $-(CF_2)_n-O-(CF_2)_p-$ which may be perfluorinated and in which n is from 1 to 5 and p is from 1 to 5, or a polyoxaalkylene radical which may be perfluorinated and has 2 to 5 oxygen atoms, which are bonded to one another via one or more $-CH_2-$ or $-CF_2-$ groups, and Y is H or methyl.

2. A process for the preparation of a compound of the formula (I), wherein a compound of the formula (II)

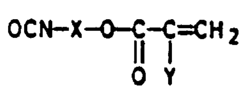

(II),

where X and Y have the meanings stated in claim 1, is reacted with a compound of the formula (III)

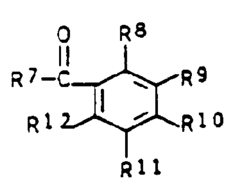

(III),

where $R^7$ is a straight-chain alkyl radical of 1 to 4 carbon atoms, an unsubstituted or substituted, branched alkyl radical of 3 or 4 carbon atoms or aryl, and $R^8$ to $R^{12}$ are identical or different and are each H, alkyl of 1 to 3 carbon atoms, OH, $OCH_3$, $OC_2H_5$, SH, $SCH_3$, Cl, F, $N(CH_3)_2$, $N(C_2H_5)_2$ or $N(CH_3)C_6H_5$, with the proviso that one or more of the radicals $R^8$ to $R^{12}$ are hydroxyl, in an equimolar ratio or in two or three times this ratio, in the presence or absence of an inert solvent or solvent mixture and of a basic catalyst, at from 0 to 100°C under anhydrous conditions.

3. A process as claimed in claim 2, wherein the compound of the formula (II) is an ω-isocyanatoalkyl (meth)-acrylate.

4. A process as claimed in claim 2 or 3, wherein from 1 to 1.2 moles of the isocyanate of the formula (II) are reacted per mole of the compound of the formula (III).

5. A process as claimed in any of claims 2 to 4, wherein not less than a catalytic amount of a strong, nonnucleophilic base, preferably of an amine, is present.

6. A process as claimed in any of claims 2 to 5, wherein the reaction temperature is from 20 to 50°C.

7. A process as claimed in any of claims 2 to 6, wherein the procedure is carried out in an inert, anhydrous solvent in the absence of moisture.

## Revendications

1. Composés organiques à insaturation éthylénique copolymérisables, sensibles aux rayonnements, de formule générale I

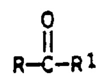

(I),

dans laquelle

R représente un groupe alkyle à chaîne droite en C 1-C 4, un groupe alkyle ramifié, éventuellement substitué, en C 3 ou C 4, un groupe aryle ou le groupe $R^1$,et $R^1$ représente le groupe

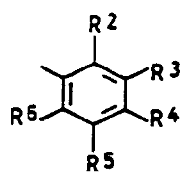

dans lequel les symboles $R^2$ à $R^6$, ayant des significations identiques ou différentes, représentent chacun H, un groupe alkyle en C 1-C 3, OH, $OCH_3$, $OC_2H_5$, SH, $SCH_3$, $SC_2H_5$, le chlore, le fluor, un groupe $N(CH_3)_2$, $N(C_2H_5)_2$ ou $N(CH_3)C_6H_5$ et au moins un mais au maximum trois des symboles $R^2$ à $R^6$ représentent le groupe

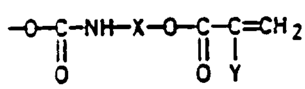

dans lequel X représente un groupe alkylène divalent éventuellement perfluoré $-(CH_2)_m-$ ou $-(CF_2)_m-$ avec m = 1 à 10, un groupe oxa-alkylène éventuellement perfluoré, du type $-(CH_2)n-O-(CH_2)_p-$ ou $-(CF_2)_p-$ avec n = 1 à 5 et p = 1 à 5, ou bien un groupe polyoxa-alkylène éventuellement perfluoré contenant 2 à 5 atomes d'oxygène reliés entre eux par au moins un groupe $-CH_2-$ou $-CF_2-$ et Y représente H ou un groupe méthyle.

2. Procédé de préparation des composés de formule générale I, caractérisé en ce que l'on fait réagir entre eux, éventuellement en présence d'un solvant ou mélange solvant inerte et d'un catalyseur basique, à des températures de 0 à 100 degrés C, en milieu anhydre, un composé de formule générale II

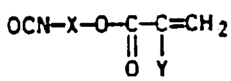

(II),

dans laquelle X et Y ont les significations indiquées dans la revendication 1, et un composé de formule générale

III

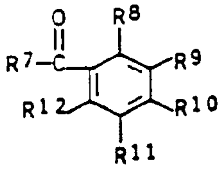

(III),

dans laquelle $R^7$ représente un groupe alkyle à chaîne droite en C 1-C 4, un groupe alkyle ramifié éventuellement substitué en C 3 ou C 4 ou un groupe aryle et les symboles $R^8$ à $R^{12}$, identiques ou différents, représentent chacun H, un groupe alkyle en C 1-C 3, OH, $OCH_3$, $OC_2H_5$, SH, $SCH_3$, le chlore, le fluor, un groupe $N(CH_3)_2$, $N(C_2H_5)_2$ ou $N(CH_3)C_6H_5$ sous réserve que l'un au moins des symboles $R^8$ à $R^{12}$ représente un groupe hydroxy, dans des proportions équimoléculaires ou doubles ou triples des proportions équimoléculaires.

3. Procédé selon la revendication 2, caractérisé en ce que les composés de formule II sont des (méth)acrylates d'oméga-isocyanatoalkyle.

4. Procédé selon l'une des revendications 2 ou 3, caractérisé en ce que, pour 1 mol du composé de formule générale III, on fait réagir 1 à 1,2 mol de l'isocyanate de formule générale II.

5. Procédé selon l'une des revendications 2 à 4, caractérisé en ce que l'on opère en présence d'une quantité au moins catalytique d'une base forte non nucléophile, de préférence une amine.

6. Procédé selon l'une des revendications 2 à 5, caractérisé en ce que la température de réaction se situe dans l'intervalle de 20 à 50 degrés C.

7. Procédé selon l'une des revendications 2 à 6, caractérisé en ce que l'on opère dans un solvant inerte anhydre, à l'abri de l'humidité.